# EUROPEAN PATENT SPECIFICATION

(11) **EP 2 448 968 B1**
(45) Date of publication and mention of the grant of the patent: **27.01.2021**
(21) Application number: 10739701.0
(22) Date of filing: 29.06.2010
(51) Int. Cl.: C07K 16/18, C07K 14/47, A61K 38/17, A61K 38/00, A61P 25/28, A61P 37/04, A61P 43/00

(54) **ANTIBODIES SELECTIVE FOR N-TERMINALTRUNCATED AMYLOID-p PROTOFIBRILS/OLIGOMERS**
SELEKTIVE ANTIKÖRPER FÜR N-TERMINAL TRUNKIERTE AMYLOID-BETA-PROTOFIBRILLEN/OLIGOMERE
ANTICORPS SÉLECTIFS POUR DES PROTOFIBRILLES/OLIGOMÈRES À BÊTA-AMYLOÏDES TRONQUÉS À LEUR EXTRÉMITÉ N-TERMINALE

(30) Priority: 29.06.2009 US 221105 P
(43) Date of publication of application: 09.05.2012
(73) Proprietor: BioArctic AB, 112 51 Stockholm (SE)
(72) Inventor: GELLERFORS, Pär, S-181 63 Stockholm (SE); LANNFELT, Lars, S-116 43 Stockholm (SE); SÖDERBERG, Linda, S-117 58 Stockholm (SE); TEGERSTEDT, Karin, S-117 39 Stockholm (SE)
(74) Representative: AWA Sweden AB
(86) International application number: PCT/IB2010/052947
(87) International publication number: WO 2011/001366

(56) References cited:
- WO-A2-01/10900
- WO-A2-2004/013172
- WO-A2-2009/133521
- US-A1- 2005 175 626
- US-B1- 7 122 374
- YOUSSEF I ET AL: "N-truncated amyloid-beta oligomers induce learning impairment and neuronal apoptosis" NEUROBIOLOGY OF AGING, TARRYTOWN, NY, US LNKD- DOI:10.1016/J.NEUROBIOLAGING.2007.03.005, vol. 29, no. 9, 1 September 2008 (2008-09-01), pages 1319-1333, XP022930369 ISSN: 0197-4580 [retrieved on 2008-07-16]
- ACERO G ET AL: "Immunodominant epitope and properties of pyroglutamate-modified Abeta-specific antibodies produced in rabbits." JOURNAL OF NEUROIMMUNOLOGY 18 AUG 2009 LNKD- PUBMED:19545911, vol. 213, no. 1-2, 18 August 2009 (2009-08-18), pages 39-46, XP002605076 ISSN: 1872-8421
- HUST MICHAEL ET AL: "Single chain Fab (scFab) fragment", BMC BIOTECHNOLOGY, BIOMED CENTRAL LTD. LONDON, GB, vol. 7, no. 1, 8 March 2007 (2007-03-08), page 14, XP021023594, ISSN: 1472-6750, DOI: 10.1186/1472-6750-7-14
- "Chapter 4.1" In: Steven A Frank: "Immnuology and Evolution of Infectious Disease", 2002, Princeton University Press, Princeton, NJ pages 35-36,
- ENGLUND H ET AL: "Sensitive ELISA detection of amyloid-beta protofibrils in biological samples", JOURNAL OF NEUROCHEMISTRY, WILEY INTERSCIENCE, NEW YORK, NY, US, vol. 103, no. 1, 1 October 2007 (2007-10-01), pages 334-345, XP002688021, ISSN: 0022-3042, DOI: 10.1111/J.1471-4159.2007.04759.X [retrieved on 2007-06-09]
- SCHILLING STEPHAN ET AL: "Glutaminyl cyclase inhibition attenuates pyroglutamate A beta and Alzheimer's disease-like pathology", NATURE MEDICINE, NATURE PUB. CO, vol. 14, no. 10, 1 October 2008 (2008-10-01), pages 1106-1111, XP002559603, ISSN: 1078-8956, DOI: 10.1038/NM.1872 [retrieved on 2008-09-28]
- Markus Morawski ET AL: "Glutaminyl cyclase in human cortex: correlation with (pGlu)-amyloid-[beta] load and cognitive decline in Alzheimer's disease", Journal of Alzheimer's disease : JAD, 1 January 2014 (2014-01-01), pages 385-400, XP55446158, Netherlands DOI: 10.3233/JAD-131535 Retrieved from the Internet: URL:https://content.iospress.com/download/ journal-of-alzheimers-disease/jad131535?id =journal-of-alzheimers-disease/jad131535 [retrieved on 2018-01-30]
- LORD A ET AL: "An amyloid-@b protofibril-selective antibody prevents amyloid formation in a mouse model of Alzheimer's disease", NEUROBIOLOGY OF DISEASE, ELSEVIER, AMSTERDAM, NL, vol. 36, no. 3, 1 December 2009 (2009-12-01), pages 425-434, XP026716877, ISSN: 0969-9961, DOI: 10.1016/J.NBD.2009.08.007 [retrieved on 2009-08-22]
- Gregory A Jicha: "Is passive immunization for Alzheimer's disease 'alive and well' or 'dead and buried'?", EXPERT OPINION ON BIOLOGICAL THERAPY, vol. 9, no. 4, 1 April 2009 (2009-04-01), pages 481-491, XP55446230, ASHLEY, LONDON; GB ISSN: 1471-2598, DOI: 10.1517/14712590902828285
- CHRISTOPHER H. VAN DYCK: "Anti-Amyloid-[beta] Monoclonal Antibodies for Alzheimer's Disease: Pitfalls and Promise", BIOLOGICAL PSYCHIATRY, vol. 83, no. 4, 1 January 2018 (2018-01-01), pages 311-319, XP55446243, NEW YORK, NY; US ISSN: 0006-3223, DOI: 10.1016/j.biopsych.2017.08.010
- YUKO HORIKOSHI ET AL: "Development of A[beta] terminal end-specific antibodies and sensitive ELISA for A[beta] variant", BIOCHEMICAL AND BIOPHYSICAL RESEARCH COMMUNICATIONS, vol. 319, no. 3, 1 July 2004 (2004-07-01), pages 733-737, XP55620547, AMSTERDAM, NL ISSN: 0006-291X, DOI: 10.1016/j.bbrc.2004.05.051

### SEQUENCE LISTING

<110> BioArtic Neuroscience AB
<120> N-TERMINAL TRUNCATED PROTOFIBRILS/OLIGOMERS FOR USE IN THERAPEUTIC AND DIAGNOSTIC METHODS FOR ALZHEIMER'S DISEASE AND RELATED DISORDERS
<130> 4007579-179961
<150> 61/221,105
   <151> 2009-06-29
<160> 12
<170> PatentIn version 3.5
<210> 1
   <211> 42
   <212> PRT
   <213> Homo sapiens
<400> 1
<210> 2
   <211> 41
   <212> PRT
   <213> Homo sapiens
<400> 2
   Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val Ile Ala
   35 40
<210> 3
   <211> 40
   <212> PRT
   <213> Homo sapiens
<400> 3
   Glu Phe Arg His Asp Ser Gly Tyr Glu Val His His Gln Lys Leu
   1 5 10 15
   Val Phe Phe Ala Glu Asp Val Gly Ser Asn Lys Gly Ala Ile Ile Gly
   20 25 30
   Leu Met Val Gly Gly Val Val Ile Ala
   35 40
<210> 4
   <211> 40
   <212> PRT
   <213> Homo sapiens
<220>
   <221> MISC_FEATURE
   <222> (1)..(1)
   <223> Glu is cyclized to pyroglutamate
<400> 4
   Glu Phe Arg His Asp Ser Gly Tyr Glu Val His His Gln Lys Leu
   1 5 10 15
   Val Phe Phe Ala Glu Asp Val Gly Ser Asn Lys Gly Ala Ile Ile Gly
   20 25 30
   Leu Met Val Gly Gly Val Val Ile Ala
   35 40
<210> 5
   <211> 40
   <212> PRT
   <213> Homo sapiens
<400> 5
   Asp Ala Glu Phe Arg His Asp Ser Gly Tyr Glu Val His His Gln
   1 5 10 15
   Lys Leu Val Phe Phe Ala Glu Asp Val Gly Ser Asn Lys Gly Ala Ile
   20 25 30
   Ile Gly Leu Met Val Gly Gly Val Val
   35 40
<210> 6
   <211> 39
   <212> PRT
   <213> Homo sapiens
<400> 6
   Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35
<210> 7
   <211> 38
   <212> PRT
   <213> Homo sapiens
<400> 7
   Glu Phe Arg His Asp Ser Gly Tyr Glu Val His His Gln Lys Leu
   1 5 10 15
   Val Phe Phe Ala Glu Asp Val Gly Ser Asn Lys Gly Ala Ile Ile Gly
   20 25 30
   Leu Met Val Gly Gly Val Val
   35
<210> 8
   <211> 38
   <212> PRT
   <213> Homo sapiens
<220>
   <221> MISC_FEATURE
   <222> (1)..(1)
   <223> Glu is cyclized to pyroglutamate
<400> 8
   Glu Phe Arg His Asp Ser Gly Tyr Glu Val His His Gln Lys Leu
   1 5 10 15
   Val Phe Phe Ala Glu Asp Val Gly Ser Asn Lys Gly Ala Ile Ile Gly
   20 25 30
   Leu Met Val Gly Gly Val Val
   35
<210> 9
   <211> 39
   <212> PRT
   <213> Homo sapiens
<400> 9
   Asp Ala Glu Phe Arg His Asp Ser Gly Tyr Glu Val His His Gln
   1 5 10 15
   Lys Leu Val Phe Phe Ala Glu Asp Val Gly Ser Asn Lys Gly Ala Ile
   20 25 30
   Ile Gly Leu Met Val Gly Gly Val
   35
<210> 10
   <211> 38
   <212> PRT
   <213> Homo sapiens
<400> 10
   Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val
   35
<210> 11
   <211> 37
   <212> PRT
   <213> Homo sapiens
<400> 11
   Glu Phe Arg His Asp Ser Gly Tyr Glu Val His His Gln Lys Leu
   1 5 10 15
   Val Phe Phe Ala Glu Asp Val Gly Ser Asn Lys Gly Ala Ile Ile Gly
   20 25 30
   Leu Met Val Gly Gly Val
   35
<210> 12
   <211> 37
   <212> PRT
   <213> Homo sapiens
<220>
   <221> MISC_FEATURE
   <222> (1)..(1)
   <223> Glu is cyclized to pyroglutamate
<400> 12
   Glu Phe Arg His Asp Ser Gly Tyr Glu Val His His Gln Lys Leu
   1 5 10 15
   Val Phe Phe Ala Glu Asp Val Gly Ser Asn Lys Gly Ala Ile Ile Gly
   20 25 30
   Leu Met Val Gly Gly Val
   35

### SEQUENCE LISTING

<110> BioArtic Neuroscience AB
<120> N-TERMINAL TRUNCATED PROTOFIBRILS/OLIGOMERS FOR USE IN THERAPEUTIC AND DIAGNOSTIC METHODS FOR ALZHEIMER'S DISEASE AND RELATED DISORDERS
<130> 4007579-179961
<150> 61/221,105
   <151> 2009-06-29
<160> 12
<170> PatentIn version 3.5
<210> 1
   <211> 43
   <212> PRT
   <213> Homo sapiens
<400> 1
<210> 2
   <211> 42
   <212> PRT
   <213> Homo sapiens
<400> 2
   Ala Glu Phe Arg His Asp Ser Gly Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala
           35                  40
<210> 3
   <211> 41
   <212> PRT
   <213> Homo sapiens
<400> 3
   Glu Phe Arg His Asp Ser Gly Gly Tyr Glu Val His His Gln Lys Leu
   1               5                   10                  15
   Val Phe Phe Ala Glu Asp Val Gly Ser Asn Lys Gly Ala Ile Ile Gly
               20                  25                  30
   Leu Met Val Gly Gly Val Val Ile Ala
           35                  40
<210> 4
   <211> 41
   <212> PRT
   <213> Homo sapiens
<220>
   <221> MISC_FEATURE
   <222> (1)..(1)
   <223> Glu is cyclized to pyroglutamate
<400> 4
   Glu Phe Arg His Asp Ser Gly Gly Tyr Glu Val His His Gln Lys Leu
   1               5                   10                  15
   Val Phe Phe Ala Glu Asp Val Gly Ser Asn Lys Gly Ala Ile Ile Gly
               20                  25                  30
   Leu Met Val Gly Gly Val Val Ile Ala
           35                  40
<210> 5
   <211> 41
   <212> PRT
   <213> Homo sapiens
<400> 5
   Asp Ala Glu Phe Arg His Asp Ser Gly Gly Tyr Glu Val His His Gln
   1               5                   10                  15
   Lys Leu Val Phe Phe Ala Glu Asp Val Gly Ser Asn Lys Gly Ala Ile
               20                  25                  30
   Ile Gly Leu Met Val Gly Gly Val Val
           35                  40
<210> 6
   <211> 40
   <212> PRT
   <213> Homo sapiens
<400> 6
   Ala Glu Phe Arg His Asp Ser Gly Gly Tyr Glu Val His His Gln Lys
   1               5                   10                  15
   Leu Val Phe Phe Ala Glu Asp Val Gly Ser Asn Lys Gly Ala Ile Ile
                   20                  25                  30
   Gly Leu Met Val Gly Gly Val Val
           35                  40
<210> 7
   <211> 39
   <212> PRT
   <213> Homo sapiens
<400> 7
   Glu Phe Arg His Asp Ser Gly Gly Tyr Glu Val His His Gln Lys Leu
   1               5                   10                  15
   Val Phe Phe Ala Glu Asp Val Gly Ser Asn Lys Gly Ala Ile Ile Gly
               20                  25                  30
   Leu Met Val Gly Gly Val Val
           35
<210> 8
   <211> 39
   <212> PRT
   <213> Homo sapiens
<220>
   <221> MISC_FEATURE
   <222> (1)..(1)
   <223> Glu is cyclized to pyroglutamate
<400> 8
   Glu Phe Arg His Asp Ser Gly Gly Tyr Glu Val His His Gln Lys Leu
   1               5                   10                  15
   Val Phe Phe Ala Glu Asp Val Gly Ser Asn Lys Gly Ala Ile Ile Gly
               20                  25                  30
   Leu Met Val Gly Gly Val Val
           35
<210> 9
   <211> 40
   <212> PRT
   <213> Homo sapiens
<400> 9
   Asp Ala Glu Phe Arg His Asp Ser Gly Gly Tyr Glu Val His His Gln
   1               5                   10                  15
   Lys Leu Val Phe Phe Ala Glu Asp Val Gly Ser Asn Lys Gly Ala Ile
               20                  25                  30
   Ile Gly Leu Met Val Gly Gly Val
           35                  40
<210> 10
   <211> 39
   <212> PRT
   <213> Homo sapiens
<400> 10
   Ala Glu Phe Arg His Asp Ser Gly Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val
           35
<210> 11
   <211> 38
   <212> PRT
   <213> Homo sapiens
<400> 11
   Glu Phe Arg His Asp Ser Gly Gly Tyr Glu Val His His Gln Lys Leu
   1               5                   10                  15
   Val Phe Phe Ala Glu Asp Val Gly Ser Asn Lys Gly Ala Ile Ile Gly
               20                  25                  30
   Leu Met Val Gly Gly Val
           35
<210> 12
   <211> 38
   <212> PRT
   <213> Homo sapiens
<220>
   <221> MISC_FEATURE
   <222> (1)..(1)
   <223> Glu is cyclized to pyroglutamate
<400> 12
   Glu Phe Arg His Asp Ser Gly Gly Tyr Glu Val His His Gln Lys Leu
   1               5                   10                  15
   Val Phe Phe Ala Glu Asp Val Gly Ser Asn Lys Gly Ala Ile Ile Gly
               20                  25                  30
   Leu Met Val Gly Gly Val
                                   35

## Description

### FIELD OF THE INVENTION

The invention is defined in the appended claims and any subject matter not falling within the scope of the claims is for information only. This invention provides antibodies selective for N-terminal truncated amyloid beta protein (Aβ) protofibrils/oligomers, which also bind N-terminal truncated Aβ monomer but exhibit no or substantially no binding to non-N-terminal truncated Aβ monomers. In one embodiment, the antibodies are of IgG class, in particular of IgG1 or IgG4 subclass or combinations thereof or mutations thereof and retain high Fc receptor binding and low C1(C1q) binding. The antibodies may be effective in clearance of Aβ protofibrils, and have reduced risk of inflammation.

### BACKGROUND OF THE INVENTION

Alzheimer's disease (AD) is a progressive and irreversible neurodegenerative disorder causing cognitive, memory and behavioural impairments. It is the most common cause of dementia in the elderly population affecting roughly 5 % of the population above 65 years and 20 % above 80 years of age. AD is characterized by an insidious onset and progressive deterioration in multiple cognitive functions. The neuropathology involves both extracellular and intracellular argyrophillic proteineous deposits. The extracellular deposits, referred to as neuritic plaques, mainly consist of amyloid beta protein (Aβ) surrounded by dystrophic neurites (swollen, distorted neuronal processes). Aβ within these extracellular deposits are fibrillar in character with a β-pleated sheet structure. Aβ in these deposits can be stained with certain dyes, e.g. Congo Red, and display a fibrillar ultra structure. These characteristics, adopted by Aβ in its fibrillar structure in neuritic plaques, are the definition of the generic term amyloid. The classic intracellular AD pathologic lesion is the neurofibrillary tangle (NFT) which consists of filamentous structures called paired helical filaments (PHFs), composed of twisted strands of hyperphosphorylated microtubule-associated protein tau. Frequent neuritic plaques and neurofibrillary tangle deposits in the brain are diagnostic criteria for AD, as carried out post mortem. AD brains also display macroscopic brain atrophy, nerve cell loss, local inflammation (microgliosis and astrocytosis) and often cerebral amyloid angiopathy (CAA) in cerebral vessel walls.

Brain pathology indicative of AD, e.g. amyloid plaques and neurofibrillary tangles, are seen in several other disorders. After the age of 30 years, almost all subjects with Down's syndrome have developed the typical neuropathological hallmarks of AD with depositions and NFT (*Zigman 1996*). Approximately 50 % of cases with Dementia with Lewy Bodies have a coexistant neuropathology indicative of AD. It is unclear whether the existence of parallel pathologies implies two different diseases or just represents a variant of each respective disorder. Sometimes the cases with co-pathology are described as having a Lewy body variant of AD (*Hansen et al., 1990*). Mixed dementia refers to a combination of AD and vascular encephalopathy, but the distinction between both disorders is controversial. For the diagnosis of mixed dementia the clinical/neuroimaging criteria of possible AD plus cerebrovascular disease as separate entities are used, but causal relations between vascular brain lesions and dementia are unclear. In a consecutive autopsy series of 1500 demented elderly subjects, 830 of which with clinically probable AD, in Vienna, Austria, 41.5 to 52.0 % showed "pure" AD, 7 % atypical AD, 16-20 % AD plus cerebrovascular lesions, and 9 % AD plus Lewy body pathology. This indicates frequent coexistence of AD with multiple cerebrovascular lesions in cognitively impaired patients (*Jellinger* 2007). US7122374 discloses an antibody that reacts with AβN3pE-42.

Glaucoma and Age-Related Macular Degeneration are the major causes of blindness world-wide. Increasing evidence support similar pathological mechanisms involving Aβ leading to loss of vision as implicated in the AD brain. In age-related macular degeneration numerous and/or confluent drusen are associated clinically with geographic atrophy of the retinal pigmented epithelium. Aβ is deposited in these drusen in human donor eyes (*Johnson 2002*)*.* A monoclonal antibody targeting a linear epitope at the C-terminus has been demonstrated to have a therapeutic potential in a mouse model of age-related macular degeneration (*Ding 2008*). Aβ has been reported to be implicated in the apoptosis of the retinal ganglion cells in experimental glaucoma (*Guo 2007*)*.* In this animal model the induced apoptosis of retinal ganglion cells can be rescued by treatment with commercial available antibodies with linear epitopes.

Inclusion body myositis is the most common acquired muscle disease in the elderly. Typically, the hallmarks of AD are found in muscle biopsies from patients with this disease: the presence of Aβ depositions, congophilic inclusions, and tau pathology (*Needham 2008*) In conclusion, Aβ deposits can be found in neurodegenerative disorders and other disorders affecting the eye and the muscles and implicated as a disease causing factor.

Two forms of Aβ peptides, Aβ40 and Aβ42, are the dominant species in AD neuritic plaques while Aβ40 is the prominent species in cerebrovascular amyloid associated with AD. Enzymatic activities allow Aβ to be continuously formed from a larger protein called the amyloid precursor protein (APP) in both healthy and AD afflicted subjects in all cells of the body. Two major APP processing events through β- and γ-secretase activities enable Aβ production, while a third enzyme called α-secretase prevents Aβ generation by cleavage inside the Aβ sequence (*Selkoe, 1994; Ester 2001;* US5604102). Aβ42 is a forty-two amino acid long peptide, i.e. two amino acids longer at the C-terminus, as compared to Aβ40. Aβ42 is more hydrophobic and more easily aggregates into larger structures of Aβ peptides (*Jarret 1993)* such as Aβ dimers, Aβ trimers, Aβ tetramers, higher Aβ oligomers such as Aβ protofibrils, or Aβ fibrils. Aβ fibrils are hydrophobic and insoluble, while the other structures are all less hydrophobic and soluble. All these higher molecular structures of Aβ peptides are individually defined based on their biophysical and structural appearance, e.g. in electron microscopy, and their biochemical characteristics, e.g. by analysis with size-exclusion chromatography/western blot. These Aβ peptides, particularly Aβ42, will gradually assemble into various higher molecular structures of Aβ during the life span. AD, which is a strongly age-dependent disorder, will occur earlier in life if this assembly process occurs more rapidly. This is the core of the "amyloid cascade hypothesis" of AD which claims that APP processing, the Aβ42 levels and their assembly into higher molecular structures is a central cause of AD. All other neuropathology of AD brain and the symptoms of AD such as dementia are somehow caused by Aβ or assembled forms thereof.

Aβ can exist in different lengths, e.g. 1-38, 1-39, 1-40, 1-41, 1-42 and 1-43 and fragments thereof in various sizes, e.g. 1-28 and 25-35. Truncations might also occur at the N-terminus of the peptide. N-terminal truncated monomeric Aβ has been characterized from insoluble Aβ extracted in pure-formic acid from AD brain tissue (*Sergeant 2003*) and CSF. They can exist in different lengths. Some forms can also be oxidized at Met35. Some of these fragments can be methylated at the N-terminus or form a pyroglutamyl residue at the N-terminus by glutaminyl cyclase (QC). The QC enzyme acts on N-terminal glutamate and glutamine causing a pyroglutamate (pE) modification at the N-terminus. One such pyroglutamate-modified Aβ fragment is the Aβ3(pE)-42 that also has been shown to have an intracellular localization. The Aβ3(pE)-42 form has also been shown to have high stability and aggregation propensity. All these peptides can aggregate and form soluble intermediates and insoluble fibrils, each molecular form having a unique structural conformation and biophysical property. Monomeric Aβ1-42 for example, is a 42 amino acid long soluble and non-toxic peptide, which is suggested to be involved in normal synapse functions. Under certain conditions, the Aβ1-42 can aggregate into dimers, trimers, tetramers, pentamers and higher oligomeric forms, all with distinct physicochemical properties such as molecular size, EM structure and AFM (atomic force microscopy) molecular shape. One example of a higher molecular weight soluble oligomeric Aβ form is the protofibril *(Walsh 1997*), which generally has an apparent molecular weight >100 kDa and with a curve linear structure of 4-11 nm in diameter and < 200 nm in length. It has been demonstrated that soluble oligomeric Aβ, such as Aβ protofibrils, impair long-term potentiation (LTP), a measure of synaptic plasticity that is thought to reflect memory formation in the hippocampus (*Walsh 2002*)*.* Furthermore, oligomeric Arctic Aβ peptides, which are mutated forms which (see below) display much more profound inhibitory effect than wild type Aβ (wtAβ<9 on LTP in the brain, likely due to their strong propensity to form Aβ protofibrils (*Klyubin* 2003)).

There are also other soluble oligomeric forms described in the literature that are distinctly different from protofibrils. One such oligomeric form is ADDL (Amyloid Derived Diffusible Ligand) *(Lambert 1998).* AFM analysis of ADDL revealed predominantly small globular species of 4.7-6.2 nm along the z-axis with molecular weights of 17-42 kDa *(Stine* 1996). Another form is called ASPD (Amyloidspheroids) (*Hoshi 2003*)*.* ASPD are spherical oligomers of Aβ1-40. Toxicity studies showed that spherical ASPD >10 nm were more toxic than lower molecular forms (*Hoshi* 2003). This idea has gained support from discovery of the Arctic (E693) APP mutation, which causes early-onset AD (US 2002/0162129 A1; Nilsberth et al., 2001). The mutation is located inside the Aβ peptide sequence. Mutation carriers will thereby generate variants of Aβ peptides e.g. Arctic Aβ40 and Arctic Aβ42. Both Arctic Aβ40 and Arctic Aβ42 will much more easily assemble into higher molecular structures i.e. protofibrils.

In the Alzheimer's disease (AD) brain, extracellular amyloid plaques are typically found in parenchyma and vessel walls. The plaques are composed of amyloid Aβ38-43 amino acid long hydrophobic and self-aggregating peptides, which gradually polymerize prior to plaque deposition. The soluble Aβ oligomeric species have been proposed to be better disease correlates than the amyloid plaques themselves (*McLean et al., 1999; Näslund et al., 2000*). Among these pre-fibrillar intermediate Aβ species, oligomeric forms have been shown to elicit adverse biological effects both *in vitro* and *in vivo* (*Walsh et al., 2002*) and may thus play a central role in disease pathogenesis. Several oligomeric Aβ species of various molecular sizes are known. Importantly, the conformation of monomeric, oligomeric and fibrillar forms of Aβ are different and can be targeted by conformational selective antibodies. The identity of the main Aβ pathogen is unclear, although some evidence suggests high-molecular weight Aβ oligomers to be especially neurotoxic (*Hoshi et al., 2003*).

Pathogenic mutations in the *amyloid precursor protein (APP)* gene, causing early onset AD have been described. One of them, the *Swedish APP* mutation (*Mullan et al.,* 1992), causes increased levels of Aβ. The *Arctic APP* mutation (*E693G*) located within the Aβ domain, was found to enhance the formation of protofibrils, large Aβ oligomers, suggesting these Aβ intermediates to be particularly pathogenic (US 2002/0162129 A1; Nilsberth et al., 2001). The identification of the *Arctic APP* mutation and the elucidation of toxic effects for Aβ protofibrils have increased the focus on Aβ oligomers in AD pathogenesis.

Active immunization as a therapeutic strategy for Alzheimer's disease was first reported by *Schenk et al. (1999).* The target for the immunization strategy was the fibrillar form of Aβ found in Alzheimer plaques. A clinical phase I / II trial of active Aβ vaccination using fibrillized Aβ as a vaccine (AN-1792) had to be halted because of the development of meningoencephalitis in a small number of patients (*Bayer et al., 2005*)*.* The side effects seen in this study were likely caused by anti-Aβ antibodies reacting against fibrillar amyloid in vessel walls. The fibrillary amyloid in CAA is in close proximity to the blood-brain-barrier (BBB) and the antigen-antibody reaction could thus generate damage to the BBB leading to infiltration of T-lymphocytes into the CNS (*Pfeifer et al., 2002; Racke et al., 2005*). Moreover, only a minority of the participating patients displayed an immune response to the Aβ vaccine. Although the study ended prematurely, it seems to imply that active Aβ immunization may be beneficial only to a subset of AD patients.

Monoclonal antibodies selective for human Aβ protofibrils have been described (WO2005/123775). The method to generate highly pure and stable human Aβ protofibrils involves the use of synthetic Aβ42 peptides with the Arctic mutation (Glu22Gly). The mutation facilitates immunization, and hybridoma screening, for Aβ protofibril selective antibodies. Importantly, these antibodies bind both wild-type Aβ protofibrils and Aβ-Arc protofibrils.

Antibodies that are selective towards other conformations of Aβ such as Aβ fibrils (O *'Nuallain 2002),* micellar Aβ (*Kayed 2003),* ADDL (*Lambert 2001)*, have been described. However, none of these are Aβ protofibril selective.

### SUMMARY OF THE INVENTION

The present invention provides an antibody, wherein the antibody is produced from stabilized protofibril comprising the N-terminal truncated Amyloid beta (Aβ) Aβ3(pE)-42, and is selected such that it (i) binds soluble protofibril comprising N-terminal truncated Aβ3(pE)-42, (ii) binds N-terminal truncated Aβ3(pE)-42 monomer, and (iii) exhibits no or substantially no binding with non-N-terminal truncated Aβ monomer, wherein:
(a) the non-N-terminal truncated Aβ monomer may or may not have a C-terminal truncation; and
(b) the stabilized protofibril is stabilized with a hydrophobic organic agent comprising a non-ionic detergent, a zwitterionic detergent, 4-hydroxy-2-nonenal (HNE), or 4-oxo-2-nonenal (ONE), and wherein the stabilized protofibril has a lower formation rate to a non-soluble aggregated form than a non-stabilized form of the protofibril.

The invention also provides a method for detecting protofibril comprising N-terminal truncated Aβ3(pE)-42 and for detecting N-terminal truncated Aβ3(pE)-42 monomer, comprising adding an antibody of the invention to a biological sample comprising or suspected of comprising the protofibril and/or monomer, and measuring a concentration of a complex formed between the antibody and the protofibril and a complex formed between the antibody and the monomer.

Also disclosed herein is a vaccine comprising N-terminal truncated Aβ protofibril/oligomer to treat and/or diagnose Alzheimer's disease and Alzheimer-related disorders.

The vaccine disclosed herein, for delaying onset of or for treatment of Alzheimer's disease or an Alzheimer-related disorder, comprises a therapeutically effective amount of a physiologically acceptable protofibril/oligomer comprising N-terminal truncated Aβ. The protofibril/oligomer may comprise a combination of full length Aβ peptide(s) and N-terminal truncated Aβ peptide(s) in various ratios. In particular, the vaccine may comprise a therapeutically effective amount of a physiologically acceptable stabilized N-terminal truncated protofibril/oligomer having a lower formation rate to a non-soluble aggregated form than a non-stabilized form of the N-terminal truncated protofibril/oligomer.

Another aspect of the disclosure is a method for delaying onset of or for treatment of Alzheimer's disease or an Alzheimer-related disorder in an individual, comprising administering to the individual a vaccine according as described herein. The vaccine described herein may be used to produce antibodies towards protofibrils/oligomers comprising N-terminal truncated Aβ, i.e. improved antibodies with more efficient binding to the protofibrils/oligomers formed *in vivo.*

The antibody of the invention may be administered to an individual in order to delay onset of or to treat Alzheimer's disease or an Alzheimer-related disorder in an individual.

Also disclosed herein is a method of producing an antibody for delaying onset of or for treatment of Alzheimer's disease or an Alzheimer-related disorder in an individual, wherein the antibody or fragment thereof binds one or more N-truncated Aβ protofibrils/oligomers, but exhibits no or substantially no cross-reactivity with full length Aβ monomers. The method comprises administering an antigen to a non-human animal and collecting antibodies formed against the antigen, the antigen comprising stabilized N-terminal truncated protofibrils/oligomers or stabilized N-terminal truncated protofibrils/oligomers having a lower formation rate to a non-soluble aggregated form than N-terminal truncated protofibrils/oligomers, which are soluble Aβ species.

Also disclosed herein are antibody compositions and vaccine compositions, comprising an antibody or a vaccine, respectively, as described above and one or more excipients, e.g. selected from the group consisting of antibacterial agents, adjuvants, buffers, salts, pH-regulators, detergents, and any combination thereof, that are pharmaceutically acceptable for human and/or veterinary use.

Also disclosed herein is a method of detecting N-terminal truncated Aβ3(pE)-42 *in vivo.* The method comprises administering to an individual suspected of carrying N-terminal truncated protofibrils/oligomers, an antibody according to the invention, the antibody being labelled with a detectable marker; and detecting the presence of any complex formed between the antibody and the soluble N-terminal truncated Aβ3(pE)-42 compound by detection of the labelled antibody.

The vaccines, antibodies, and methods disclosed herein are advantageous for diagnostic and therapeutic techniques directed to Alzheimer's disease and Alzheimer-related disorders. Additional embodiments and aspects of the invention are set forth in the Detailed Description, and additional advantages of the invention will be apparent therefrom.

### BRIEF DESCRIPTION OF THE DRAWING

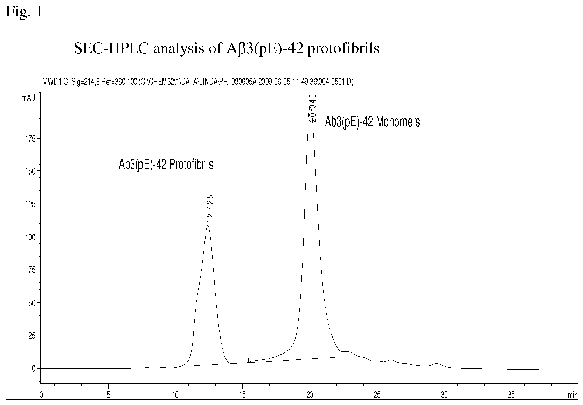

The present invention is further illustrated with reference to Fig. 1 which shows separation of Aβ3(pE)-42 monomers from Aβ3(pE)-42 protofibrils by SEC-HPLC.

### DETAILED DESCRIPTION

The present invention provides antibodies produced from stabilized protofibril comprising N-terminal truncated Aβ3(pE)-42, which are selected such that they (i) bind soluble protofibril comprising N-terminal truncated Aβ3(pE)-42, (ii) bind N-terminal truncated Aβ3(pE)-42 monomer, and (iii) exhibit no or substantially no binding with non-N-terminal truncated Aβ monomer, wherein:
(a) the non-N-terminal truncated Aβ monomer may or may not have a C-terminal truncation; and
(b) the stabilized protofibril is stabilized with a hydrophobic organic agent comprising a non-ionic detergent, a zwitterionic detergent, 4-hydroxy-2-nonenal (HNE), or 4-oxo-2-nonenal (ONE), and wherein the stabilized protofibril has a lower formation rate to a non-soluble aggregated form than a non-stabilized form of the protofibril.

Antibodies according to the invention may be used in various methods for diagnosing and combating, including delaying the onset of, treatment and/or prevention of, Alzheimer's disease and Alzheimer-related disorders.

The antibodies may be used in delaying the onset of, treatment and/or prevention of Alzheimer's disease and Alzheimer's disease-related disorders (Alzheimer-related disorders), which include without limitation, Down's syndrome, cerebrovascular amyloidosis, mixed dementia, glaucoma, age-related macular degeneration, and/or Inclusion body myositis,. These disorders might also appear in combination with other neurodegenerative disorders, e.g. alpha-synuclein related diseases.

The antibodies may also be used in detection methods for, *inter alia*, diagnostic, monitoring or therapy purposes.

The major pathology in Alzheimer's disease is extracellular toxic forms of soluble oligomeric forms of the Aβ peptide, in particular higher molecular weight forms of the oligomeric forms of Aβ, called protofibrils. Within the present disclosure, the terms "oligomer/protofibril" and "protofibril/oligomer" are used interchangeably to refer to higher molecular weight oligomers, including protofibrils. The invention is directed to an antibody that binds protofibrils/oligomers comprising N-terminal truncated Aβ3(pE)-42. Also disclosed herein is a vaccine comprising protofibrils/oligomers comprising N-terminal truncated Aβ.

Examples of N-terminal truncated Aβ peptides are those of the formula Aβx-y, wherein x is 2, 3, 3(pE), 4, 5, 6, 7, 8, 9, 10, 11, 11(pE) or 12 and y is 38, 39, 40, 41, 42 or 43, in any combination. Examples include, but are not limited to, e.g. Aβ2-40, Aβ3-40, Aβ3(pE)-40, Aβ 4-40, Aβ5-40, Aβ6-40, Aβ 7-40, Aβ 8-40, Aβ9-40, Aβ10P-40, Aβ 11-40, Aβ11(pE)-40, Aβ 12-40, Aβ2-42, Aβ3-42, Aβ3(pE)-42, Aβ 4-42, Aβ5-42, Aβ6-42, Aβ 7-42, Aβ 8-42, Aβ9-42, Aβ10-42, Aβ 11-42, Aβ11(pE)-42 and Aβ 12-42.

In Aβ3(pE)-40/42 and. Aβ11(pE)-40/42, the peptide has lost two or 10 amino acids, respectively, at its N-terminal end and the N-terminal amino acid glutamate has been cyclised to a pyroglutamyl derivative of glutamate.

At present, Aβ2-42, Aβ3(pE)-42, Aβ4-42 and/or Aβ11(pE)-42 seem to be of special importance and antibodies binding to protofibrils/oligomers comprising one of more of these in various combinations are believed to fulfil an important therapeutic role.

In one embodiment the antibody of the invention binds protofibril/oligomer comprising N-terminal truncated Aβ3(pE)-42 peptides but without full length Aβ peptides, i.e., non-N-terminal truncated Aβ peptides. Within the present specification, the terms "full length Aβ peptides" and "non-N-terminal truncated Aβ peptides" are used interchangeably and refer to Aβ peptides which do not have N-terminal truncation but may or may not have C-terminal truncation.

The antibody of the invention may also bind Aβ protofibril/oligomer containing both N-terminal truncated Aβ3(pE)-42 and non-N-terminal truncated forms of Aβ. Without being bound by theory, at present it is believed such Aβ protofibrils/oligomers provide a good estimate of a clinical situation.

The protofibril/oligomer described herein may comprise ≥ 50% N-terminal truncated Aβ peptide(s), such as ≥ 60%, ≥ 70%, ≥ 80%, ≥ 90%, or ≥ 95% or 100% of any of the N-terminal truncated peptides disclosed above, alone, or as a combination of two or more N-terminal truncated forms. The full length peptides in such a protofibril construct may be Aβ1-38, Aβ1-39, Aβ1-40, Aβ1-41, Aβ1-42 or Aβ1-43 or any combination of these. At present Aβ1-40 and in particular Aβ1-42 are believed to be the most important constituents of such a construct.

An antibody of the invention is characterized in that it exhibits high affinity for and binding to protofibrils/oligomers comprising N-terminal truncated Aβ3(pE)-42. This functionality provides important advantages compared to known antibodies raised against various species in the Aβ system. Accordingly, an antibody according to one aspect of the invention has an IC50 value ≤ 25 nM, such as ≤ 15 nM, ≤ 10 nM or even ≤ 5 nM for a protofibril/oligomer comprising at least 80 % Aβ3(pE)-42. A method for determination of IC50-values is described by Englund H. et al. in J. of Neurochemistry, 2007, 103, 334-345. The antigen (Aβ protofibrils/oligomers or monomer) concentration required to inhibit half of the maximal signal in the inhibition ELISA is defined as the IC50 value and can be used as an estimate of the antibody's affinity for the antigen. The protofibril/oligomer concentration is expressed as molarity of the monomeric subunit (∼4kD).

According to one embodiment of the invention, the antibody binds protofibril/oligomer comprising 80 % Aβ3(pE)-42 and 20 % Aβ1-42.

In one embodiment of the invention, the antibody exhibits an IC50 value ≤ 50 nM for a protofibril/oligomer comprising 90 % Aβ3(pE)-42, and in particular an IC50 value ≤ 100 nM, such as ≤ 50 nM, ≤ 25 nM, ≤ 10 nM, or even ≤ 5 nM for a protofibril/oligomer comprising 100 % Aβ3(pE)-42.

The antibody of the invention also binds N-terminal truncated Aβ3(pE)-42 monomers. A further characteristic of an antibody according to the invention is that it exhibits no or substantially no binding to non-N-terminal truncated Aβ monomers, which means no detectable binding at concentrations lower than 1 nM as measured by standard ELISA.

In yet another embodiment of the invention, the antibody binds N-terminal truncated Aβ3(pE)-42 protofibrils/oligomers comprising the Arctic mutation (E22G).

In yet another embodiment of the invention, the antibody binds N-terminal truncated Aβ3(pE)-42 protofibrils/oligomers comprising the Dutch mutation (E22Q) or the Italian mutation (E22K) or the Iowa mutation (D23N) or the Flemish mutation (A21G), or combinations of two or more of these mutations.

In yet another embodiment of the invention, the antibody binds N-terminal truncated Aβ3(pE)-42 protofibrils/oligomers comprising any combination of two or more of the Arctic, Dutch, Italian, Iowa and Flemish mutations.

To lower the levels of these N-terminal truncated Aβ protofibrils/oligomers poses a challenge to the immunotherapeutic approach. However, it is likely that a fraction of actively induced or passively administrated antibodies can bind their target and reduce it by a microglial phagocytotic process in the brain.

Also disclosed herein are methods for production of stabilized N-terminal truncated Aβ protofibrils/oligomers which considerably facilitate the design and development of antibodies according to the invention. The molecular weight of human Aβ monomers is approximately 4 kDa. Two or more Aβ monomers can aggregate and form soluble N-terminal truncated protofibrils/oligomers with a wide range of molecular weights. A dominating oligomer is generally referred to as a protofibril. However, these N-terminal truncated Aβ protofibrils/oligomers are instable and polymerize spontaneously to insoluble fibrils. Disclosed herein are methods to stabilize N-terminal truncated Aβ protofibrils/oligomers and isolate the stabilized N-terminal truncated Aβ protofibrils/oligomers, preferably in highly purified form, for antibody and vaccine development. The stabilized N-terminal truncated Aβ protofibrils/oligomers produced according to the methods disclosed herein exhibit a lower formation rate to a non-soluble aggregated form, i.e., fibrils, than non-stabilized N-terminal truncated Aβ protofibrils/oligomers. These forms are of particular interest since they exhibit a high toxicity.

In addition, N-terminal truncated forms of Aβ can be made by recombinant technology or solid phase peptide synthesis. Furthermore, N-terminal truncated Aβ protofibrils/oligomers can be derived directly from brain extracts in *post mortem* autopsied human brain tissue from cases with Alzheimer's disease. Preparations of N-terminal truncated Aβ protofibrils/oligomers produced from N-terminal truncated peptides are purified in different physiological buffers. These sample preparations are either injected as such in mice, or fractionated by chromatographic methods before injection in mice for monoclonal antibody development. The antibodies generated may be used after humanization to treat patients in a passive vaccination scheme or in diagnostic immunoassays as described in further detail herein.

Furthermore, N-terminal truncated Aβ protofibrils/oligomers, to be used for immunization of mice and monoclonal antibody development, might also be isolated from biological tissues or fluids such as blood, cerebrospinal fluid, urine or saliva from healthy individuals or patients with Alzheimer's disease and/or an Alzheimer-related disorder. The stabilization of N-terminal truncated Aβ protofibrils/oligomers, may be accomplished in various ways, such as for example structural modification. The structural modification may be achieved by binding to a stabilizing agent. The binding may be in the form of cross-linking. The stabilizing agent may in particular be a hydrophobic organic agent. The hydrophobic organic agent may comprise a saturated, unsaturated, or polyunsaturated fatty acid, or derivative thereof, or any combination thereof, e.g., a combination of any two or more thereof. The hydrophobic organic agent may alternatively comprise a reactive aldehyde. The aldehyde may, for example, be an alkenal, such as an α,β-unsaturated aldehyde. Suitable reactive aldehydes include, but are not limited to, 4-hydroxy-2-nonenal (HNE), 4-oxo-2-nonenal (ONE), malondialdehyde and acrolein. The aldehyde may also be a dialdehyde having a mono or polyunsaturated carbon chain of 2-25 carbon atoms connecting the aldehyde groups. The hydrophobic organic agent stabilizes the N-terminal truncated Aβ protofibril/oligomer conformation, such that further aggregation to the non-soluble fibril conformation is prevented.

N-terminal truncated Aβ protofibrils/oligomers can alternatively be modified by hydrophobic detergents such as, but not limited to, non-ionic and zwitterionic detergents. Examples of such detergents include, but are not limited to, non-ionic detergents such as Triton X-100 (polyethylene glycol p-(1,1,3,3-tetramethylbutyl)-phenylether), Tween-20 (Polyoxyethylene (20) sorbitan monolaurate), Tween-80 (Polyoxyethylene (20) sorbitan monooleate), and Brij detergents (Polyoxyethylene ethers of fatty alcohols), and zwitterionic detergents such as CHAPS (3-[(3-cholamidopropyl)dimethylammonio]-1-propanesulfonate).

Other suitable stabilizing agents include bile acid derivatives, examples of which include, but are not limited to, cholate, deoxycholate and taurocholate, and any combination thereof.

The stabilizing agent can also be selected from the group of natural biological molecules, examples of which include, but are not limited to, triglycerides, phospholipids, sphingolipids, gangliosides, cholesterol, cholesterol-esters, long chain (for example containing about 6 to 30 carbon atoms) alcohols, and any combinations of the above agents.

Stabilization may also be accomplished using protein cross-linking agents such as, but not limited to, disuccinimidyl tartrate, bis-sulfosuccinimidyl suberimidate, 3,3-dithiobis-sulfosuccinimidyl propionate, and any combination thereof.

tabilized N-terminal truncated Aβ protofibrils/oligomers may alternatively comprise 1-alpha-hydroxy-secosterol as a stabilizing agent.

The antibody according to the invention is produced from protofibril stabilised with a hydrophobic organic agent comprising a non-ionic detergent, a zwitterionic detergent, 4-hydroxy-2-nonenal (HNE), or 4-oxo-2-nonenal (ONE).

The stabilizing agents may be bound to, including by cross-linking, monomers and/or protofibrils/oligomers of N-terminal truncated Aβ, or combination thereof, to form the stabilized N-terminal truncated Aβ protofibrils/oligomers. For example, reactive aldehyde based on non-saturated fatty acids, such as HNE and ONE, may bind to the oligomers by way of the aldehyde group or a double bond, or both. The latter then results in cross-linkage of the oligomers. HNE for example, may bind covalently to histidines and lysines of the oligomers. Similarly, ONE may bind covalently to histidines and lysines. The aldehydes may bind to lysine via a Shiff's base, or a histidine may bind via a nucleophilic attack on the carbon atom of a double bond in an unsaturated carbon chain. The stoichiometry between the stabilizing agent, for example, a reactive aldehyde, such as HNE and ONE, and N-terminal truncated Aβ peptide can be varied within a wide range of 2:1 to 50:1 or higher. HNE modification values above 20:1, e.g. 25:1, 30:1, 35:1, 40:1, 45:1, 50:1, and even higher, may provide a product with desirably high protofibril formation. In addition, with ONE, an even lower ratio can be used, e.g. from 5:1, 10:1, 15:1, 20:1, 25:1, 30:1, 35:1, 40:1, 45:1, 50:1, and even higher.

If not stated otherwise, all of the stabilizing agents mentioned above impart their stabilizing effect by binding to N-terminal truncated Aβ and the stabilizing reaction may be conducted, for example, by incubation, as illustrated in the examples. The stabilizing agents may also be used in combinations of two or more as desired.

The stabilized N-terminal truncated Aβ protofibrils/oligomers may include one or more of the following proteins: α-synuclein, tau or phospho-tau, in any combination. These mixtures are advantageous in that the additional components are found in patients with dementia, for example, but not limited to, Alzheimer's disease, but also the Lewy body variant of Alzheimer's disease, and hence will provide therapeutically important neo-epitopes for antibody or vaccine treatment of these disorders (Tsigelny et al 2008). Another advantage is that the additional components will increase stability of N-terminal truncated Aβ protofibrils /oligomers.

Disclosed herein is a vaccine for delaying onset of or for treatment of Alzheimer's disease and/or Alzheimer-related disorders. In the present disclosure, the term vaccine is used to refer to a composition which is in a physiologically acceptable form for human or animal administration for active immunization. The vaccine comprises a therapeutically effective amount of N-terminal truncated Aβ protofibrils/oligomers, e.g. isolated N-terminal truncated Aβ protofibrils/oligomers. The term isolated refers to the N-terminal truncated Aβ protofibrils/oligomers having been separated from preparation media, reactants and the like, including soluble N-terminal truncated Aβ peptides. The N-terminal truncated Aβ protofibrils/oligomers may be stabilized N-terminal truncated Aβ protofibrils/oligomers as discussed above, having been separated from preparation media, reactants and the like, including soluble N-terminal truncated Aβ peptides. The vaccine may comprise from about 10-500 microgram/dose of the N-terminal truncated Aβ protofibrils/oligomers or stabilized N-terminal truncated Aβ protofibrils/oligomers. In particular, the vaccine may comprise from about 50-250 microgram/dose of the N-terminal truncated Aβ protofibrils/oligomers or stabilized N-terminal truncated Aβ protofibrils/oligomers.

The vaccine for active immunization may comprise one or more excipients as conventionally employed in the vaccine art, examples of which include, but are not limited to, one or more antibacterial agents, adjuvants, buffers, salts, pH-regulators, detergents, or a combination thereof, provided the excipients are pharmaceutically acceptable for human and veterinary use. The vaccine may also be freeze-dried, e.g. together with one or more excipients to increase stability of the vaccine during and/or after freeze-drying. Specific examples of suitable excipients include, but are not limited to, mannitol and/or trehalose.

The invention is directed to antibodies that bind soluble protofibrils/oligomers comprising N-terminal truncated Aβ3(pE)-42, and N-terminal truncated Aβ3(pE)-42 monomers, but which exhibit no or substantially no binding with non-N-terminal truncated Aβ monomer. N-terminal truncated Aβ3(pE)-42 protofibrils/oligomers stabilised with a hydrophobic organic agent comprising a non-ionic detergent, a zwitterionic detergent, 4-hydroxy-2-nonenal (HNE), or 4-oxo-2-nonenal (ONE), and having a lower formation rate to a non-soluble aggregated form than a non-stabilized form of the protofibril, are used as an antigen to produce such antibodies, and may be used to optimize the development of specific antibodies against toxic N-terminal truncated Aβ3(pE)-42 protofibrils. In such methods, the antigen is administered to a non-human animal and the antibodies produced against said antigen are collected. In order to maximize the therapeutic effect, the antibodies according to the present invention, raised against the stabilised N-terminal truncated Aβ(pE)-42 protofibril/oligomer antigen, advantageously also exhibit high binding to natural forms of N-terminal truncated Aβ protofibrils/oligomers, and preferably also to N-terminal truncated monomers present in the body, in particular the aggregated soluble forms of Aβ. The antibodies exhibit no or substantially no binding to full length monomers, in particular Aβ1-42 monomers and/or the Amyloid Precursor Protein (APP) and/or other amyloids. One way of selecting such antibodies is to do so in steps with a first screen for antibodies that bind to the N-terminal truncated Aβ3(pE)-42 protofibril/oligomer, and among these select for species which fulfill the other required characteristics, including having substantially no binding to full length Aβ monomers, in particular Aβ1-42 monomers, APP and other amyloids, and subsequently, to screen among these antibodies for antibodies that bind well to wild type human N-terminal truncated Aβ protofibrils or a mixture of protofibrils which reflects an *in vivo* situation.

A protofibril/oligomer as defined herein may be used for the manufacture of a pharmaceutical composition for delaying onset of or for treatment of Alzheimer's disease or an Alzheimer-related disorder.

An antibody as defined herein may also be used for the manufacture of a pharmaceutical composition for delaying onset of or for treatment of Alzheimer's disease or an Alzheimer-related disorder.

Also disclosed herein are antibodies that bind both N-terminal truncated Aβ protofibrils/oligomers and Aβ protofibrils/oligomers comprising mainly non-N-terminal truncated Aβ peptides. Such antibodies are advantageous in lowering both of these Aβ protofibril/oligomer forms, as well as protofibrils comprising a mix of full length Aβ and N-terminal truncated Aβ in the brain, by using one antibody. Accordingly, the disclosure includes a bifunctional antibody recognizing two epitopes in the Aβ system. Such a bifunctional antibody may be used to delay onset of and/or for treatment of Alzheimer's disease or an Alzheimer-related disorder.

Alternatively, an antibody specific for N-terminal truncated Aβ protofibrils/oligomers, and optionally also N-terminal truncated monomers, can be used in therapy in combination with an antibody that binds non-N-terminal truncated Aβ protofibrils/oligomers (non-truncated), thereby achieving a therapeutic important reduction of both N-terminal truncated Aβ protofibrils/oligomers and Aβ protofibrils/oligomers (non-truncated). Disclosed herein is a method for delaying onset and/or treatment of Alzheimer's disease or Alzheimer-related diseases by administration of such a combination of antibodies. Such administration can be made simultaneously using a single pharmaceutical preparation containing both antibodies or as two compositions, or with two compositions also by sequential administration.

The resulting antibodies may be monoclonal or polyclonal antibodies, or active fragments thereof that bind N-terminal truncated Aβ protofibrils/oligomers before Aβ has aggregated to Aβ fibrils and could potentially also reduce the amount of fibrils already formed. The N-terminal truncated Aβ protofibril antigen may be used in methods such as hybridoma technology, phage display, ribosome display, mammalian cell display, and bacterial display, for producing and/or evolving monoclonal or polyclonal antibodies, or active fragments thereof. More specifically, for generation of monoclonal antibodies, a conventional technique, such as the hybridoma technique and/or phage display, ribosome display, mammalian cell display, or bacterial display may be employed. Such antibodies may be produced in rodents such as mouse, hamster or rat. Once generated, clones are isolated and screened for their respective antigen specificity. For screening, two principles are used. Firstly, antibodies are probed against purified N-terminal truncated Aβ monomers, N-terminal truncated Aβ protofibrils/oligomers and fibrils. These different conformational forms of N-terminal truncated Aβ can be made by incubating N-terminal truncated Aβ peptide or stabilized N-terminal truncated Aβ protofibrils, for example, HNE-modified and/or ONE-modified, and subsequently fractionating by HPLC or using a centrifugal filter device. N-terminal truncated Aβ fibrils can be isolated by centrifugation of the incubation mixture (Example 1). Screening may be done by an enzyme-linked immunosorbent assay (ELISA) or by similar methods. Secondly, the antibodies are evaluated on tissue slices from transgenic animals and/or pathologic Alzheimer human brain tissue sections.

The antibody of the invention reacts with both stabilized, as well as non-stabilized, human N-terminal truncated Aβ3(pE)-42 protofibrils/oligomers, in mutated or wild type form but with no or substantially no binding to full length Aβ monomeric forms, in particular Aβ1-42, APP or any other amyloid.

The antibody of the invention may be human, humanized, or modified to reduce antigenicity in humans according to methods known by those skilled in the art. The reduction of antigenicity may for example be made by modifying or eliminating the T-cell epitopes of the antibody. In one embodiment, the antibody is selected from the IgG class, or more preferably from the IgG1 or IgG4 subclass (human antibody).

In additional embodiments, the antibody may also have reduced complement activity and/or altered Fc receptor binding properties. This may, for example, be achieved by mutating the Fc part of the antibody in positions 297, 322 or 331 of the amino acid sequence of the heavy chain (human), or the corresponding amino acids in, for example, mouse IgG.
(Duncan & Winter, Nature 1988, 332: 738-470 and Idusogie et al, Journal of Immunology, 2000, 164: 4178-84). The reduced complement activity may also be achieved by deglycosylating the antibody enzymatically or by other means, in accordance with techniques known in the art. Altered Fc receptor binding properties of the antibody may be achieved by altering the oligosaccharide structures attached to the glycoprotein (Jeffries, Nature, 2009, 8: 226-234). The antibody may be a Fab fragment, for example selected from F(ab), F(ab)2, and DiFabody, or a single chain antibody, for example selected from scFv-Fc and scFab, e.g. to improve blood brain barrier penetrance and neuronal cell uptake. It is therefore apparent that antibody as used herein refers to a full length protein raised by the antigen or an active fragment thereof.

N-terminal truncated Aβ protofibril/oligomer antigen may be fractionated and isolated by SEC-HPLC. The fractions are assessed for their respective toxicity in cell culture models and the antigen fractions with the strongest toxicity are selected as antigens for antibody production or as antigens for active immunization. The sample preparations can also be used directly to assess toxicity and the samples showing the most pronounced toxicity may advantageously be used as antigen for antibody selection and/or production or as antigens for active immunization.

The N-terminal truncated Aβ protofibril/oligomer antibodies are formed as a response to administering the N-terminal truncated Aβ protofibrils/oligomers described herein, either directly to the patient (active immunization) or by immunizing a rodent, for example a mouse or a rabbit, in order to raise monoclonal or polyclonal antibodies against the antigen, which are applied in a passive immunization protocol to treat neurodegenerative disorders such as Alzheimer's disease and Alzheimer-related disorders. In the case of passive immunization, the antibodies may be humanized before being administered to the patient.

Following an active immunization protocol, the selected N-terminal truncated Aβ protofibril/oligomer antigen is administered to yield conformation-specific antibodies directed towards N-terminal truncated Aβ protofibrils/oligomers species with pronounced toxicity, in particular protofibrillar/oligomeric species. Following a passive immunization protocol, monoclonal or polyclonal antibodies against such N-terminal truncated Aβ protofibril/oligomer species exert their effect upon repeated injections of the antibodies.

The antibody which binds N-terminal truncated Aβ protofibrils/oligomers may be a human antibody derived from white blood cells from control human subjects or patients with Alzheimer's disease. Hybridomas are made from the white blood cells according to established techniques and screened for binders to N-terminal truncated Aβ protofibrils/monomers and stabilized N-terminal truncated Aβ protofibrils/oligomers. Human monoclonal antibodies against N-terminal truncated Aβ protofibrils/oligomers can also be obtained by screening a human antibody library for binding to N-terminal truncated Aβ protofibrils/monomers. Autoantibodies against N-terminal truncated Aβ protofibrils/oligomers present in blood from human control subjects or patients with Alzheimer's or an Alzheimer-related disease may also be isolated for use. Said autoantibodies can be sequenced and made by recombinant DNA technology in, for example, CHO cells to improve yield and economy.

The antibody as described may be provided in a composition, for example, suitable for administration. Such compositions may comprise an antibody as described herein and one or more excipients conventionally employed in pharmaceutical compositions. The antibody will be included in a therapeutically effective amount. The compositions may comprise the antibody in an amount of about 0.1-5 mg/kg, or more specifically, about 0.5-2 mg/kg, of body weight of the intended recipient.

Suitable excipients include, but are not limited to, one or more antibacterial agents, adjuvants, buffers, salts, pH-regulators, detergents, or any combination thereof, provided that such excipients are pharmaceutically acceptable for human and/or veterinary use. The composition may be freeze-dried, for example together with an excipient to increase stability of the antibody during and/or after freeze-drying. Mannitol and/or trehalose are non-limiting examples of excipients suitable for the freeze-drying.

The vaccines and antibodies as described herein may be used in one or more methods for preventing, delaying onset of, or treating Alzheimer's disease or an Alzheimer-related disorder in an individual. Such methods comprise administering an antibody or vaccine as described herein to the individual. The individual is, for example, a subject suspected of having acquired or having an increased risk of acquiring Alzheimer's disease.

A subject could be suspected of having such a disorder by displaying any of the following characteristics: early disease symptoms, positive brain imaging results and increased levels of tau or P-tau. Examples of brain imaging methods include, but are not limited to DaTscan (¹²³I-Ioflupane), or Positron Emission Tomography (PET) imaging by using a monoclonal antibody as described herein.

By identifying subjects at risk of or suspected of having Alzheimer's disease or Alzheimer-related disorder, further development of the disorder is prevented or onset or progression is delayed by the inventive treatments described herein, e.g. by using active or passive immunization with the vaccines/antigens or antibodies.

The antibodies of the invention as described herein may also be used in detection methods, a specific example of which includes diagnostic immunoassays in which the antibodies are used to detect altered levels of N-terminal truncated Aβ3(pE)-42 protofibrils/oligomers and/or N-terminal truncated Aβ3(pE)-42 monomer species *in vitro* and *in vivo.* The targeted forms of N-terminal truncated Aβ protofibrils/oligomers may serve as early biochemical markers for Alzheimer's disease and Alzheimer-related disorders, in particular those mentioned above.

More specifically, a method of detecting soluble N-terminal truncated Aβ3(pE)-42 protofibrils/oligomers *in vitro* comprises adding the antibody according to the invention to a biological sample comprising or suspected of comprising N-terminal truncated Aβ3(pE)-42 protofibrils/oligomers, and detecting and measuring a concentration of any complex formed between the antibody and N-terminal truncated Aβ3(pE)-42 protofibrils/oligomers. The biological sample may be, for example, plasma, cerebrospinal fluid (CSF) or a brain biopsy. Also disclosed herein is a method of detecting N-terminal truncated Aβ3(pE)-42 protofibrils/oligomers *in vivo*, comprising administering an antibody according to the present invention, said antibody being labelled with a detectable marker, to an individual suspected of carrying unhealthy N-terminal truncated Aβ3(pE)-42 protofibrils/oligomers in the brain, and detecting the presence of any complex formed between the antibody and N-terminal truncated Aβ3(pE)-42 protofibrils/oligomers by detection of the marker.

For labelling of the antibodies against N-terminal truncated Aβ species, one of ordinary skill in the art has access to various alternatives, depending on the choice of detection method, e.g. radioactive ligands such as ¹³¹I, ¹⁴C, ³H or ⁵⁸Ga, just to mention a few. In particular, PET with a radiolabeled oligomer-specific antibody is believed to be of great importance for diagnosis, therapy monitoring, and/or the like. Accordingly, the invention provides antibodies that are easily labelled by one of ordinary skill in the art, for use in various methods for diagnosis and therapy monitoring.

In accordance with the methods and techniques described, the antigens and antibodies described herein may be evaluated for their therapeutic potential in cell culture models and/or transgenic animal models for Alzheimer pathology.

The antibodies against N-terminal truncated Aβ3(pE)-42 according to the invention are also utilized in immunobased assays for the measurement of N-terminal truncated Aβ3(pE)-42 levels in patient samples to diagnose Alzheimer's disease. The detection methods applied in the diagnostic assay are mainly based on immunoassays, such as enzyme-linked immunosorbent assay (ELISA) and/or Western blot. A broad range of tissues from patients with early signs of Alzheimer's disease or individuals with a high risk of developing these disorders are investigated for their levels of N-terminal truncated Aβ3(pE)-42 protofibril /oligomer/monomer, such tissues include, but are not limited to, plasma, cerebrospinal fluid (CSF) and brain biopsies.

### EXAMPLES

Various aspects of the disclosure are illustrated in the following Examples.

### Example 1. Preparation of Aβ3(pE)-42 truncated protofibrils/oligomers.

Lyophilized Aβ3(pE)-42 is dissolved in 10 mM NaOH to a final concentration of 100 µM. Phosphate-buffer saline, pH 7.4 is added to a final peptide concentration of 50 µM. The peptide solution is incubated at room temperature or 37°C for 5-15 min. The incubation time for optimal protofibril yield is determined in a small-scale pilot kinetic experiment, prior to the set-up of a larger reaction volume, since the aggregation process is very different from lot to lot and also from one vial to another. After incubation, the sample is centrifuged at 16 000 x g for 5 minutes at +4°C to remove fibrillar material which then is pelleted. The supernatant contains protofibrils/oligomers and various amounts of Aβ3(pE)-42 monomers. One-hundred µl of the supernatant is injected to a Superdex 75 column equilibrated with PBS containing 0.1-0.6 % Tween-20. The separation of protofibrils from monomers is carried out at a flow rate of 80µl/min. The UV absorbance is monitored by wavelength 214 and 280 nm. The void peak eluting at 12-13 min, contains Aβ3(pE)-42 protofibril/oligomer (See Fig 1). Highly pure Aβ3(pE)-42 protofibrils/oligomers for monoclonal and /or vaccine development is isolated by collecting fractions eluting at 12-13 minutes.

### Example 2. Preparation of Aβ3-42 truncated protofibrils/oligomers.

Lyophylized Aβ3-42 is dissolved in 10 mM NaOH to a final concentration of 100 µM. Phosphate-buffer saline, pH 7.4 is added to a final peptide concentration of 50 µM. The peptide solution is incubated at room temperature or 37°C for 5-15 min. The incubation time for optimal protofibril yield is determined in a small-scale pilot kinetic experiment, prior to the set-up of a larger reaction volume, since the aggregation process is very different from lot to lot and also from one vial to another. After incubation at +37°C, the sample is centrifuged at 16 000 x g for 5 minutes at +4°C to remove fibrillar material which then is pelleted. The supernatant contains protofibrils/oligomers and various amounts of Aβ3-42 monomers. One-hundred µl of the supernatant is injected to a Superdex 75 column equilibrated with PBS containing 0.1-0.6% Tween-20. The separation of protofibrils/oligomers from monomers is carried out at a flow rate of 80 µl/min. The UV absorbance is monitored by wavelength 214 and 280 nm. The void peak eluting at 12-13 min, contains Aβ3-42 protofibril/oligomer. Highly pure Aβ3-42 protofibrils/oligomers for monoclonal and /or vaccine development is isolated by collecting fractions eluting at 12-13 minutes.

### Example 3. Synthesis of stabilized Aβ3(pE)-42 truncated protofibrils/oligomers.

To produce N-terminal truncated Aβ protofibril antigen (i.e., antigen containing protofibrils and other oligomers), the human wild type N-terminal truncated Aβ3(pE)-42, as described above, is used in a concentration of 35-750 µM. In samples in which Aβ3(pE)-42 has been conjugated to HNE and/or ONE (Cayman Chemical, Ann Arbor, MI, USA), these compounds are used at a concentration of 0.01-65 mM. In a typical experiment, the molar ratio between HNE and/or ONE and Aβ3(pE)-42 ranges between 1:1 and 100:1, but the proportion of the respective compounds is not limited to this stoichiometry. In certain experiments, sodium borohydride (NaBH₄) is used at concentration of 0,1-100 mM to reduce the HNE-modified and/or ONE-modified samples. In some cases, the Aβ3(pE)-42 amino acid may contain amino acids (such as lysine) that during said HNE-modification forms an unstable and reversible Shiff's base that binds with HNE. In another case, the Aβ3(pE)-42 amino acid may contain amino acids (such as lysine) that during said ONE-modification forms an unstable and reversible Shiff's base that binds with ONE. The sodium borohydride reduction stabilizes said Shiff's base binding. The samples are incubated at 37°C with or without agitation for 30 minutes to 30 days. To verify the molecular composition of the samples, several methods are utilized. Unmodified Aβ3(pE)-42 or HNE-modified and/or ONE-modified Aβ3(pE)-42 or Aβ3(pE)-42 modified with other reactive aldehydes, are centrifuged at 16.900 x g for five min. at 21°C to remove any insoluble fibrils. The supernatant is subsequently fractionated using a SEC-HPLC system with UV detection between 214 nm and 280 nm (described in detail below) to isolate Aβ3(pE)-42 protofibril/oligomer and monomers. In another experiment, Aβ3(pE)-42 protofibrils/oligomers and monomers are separated using a centrifugal filter device with a molecular cut-off between 5-1000 kDa. In a typical experiment, samples, 500 µl HNE and/or ONE-modified Aβp3-42, are centrifuged using either a Microcon centrifugal filter device (Millipore, Billerica, MA) or a Vivaspin500 centrifugal device (Sartorius, Goettingen, Germany) with a cut-off value of 100 kDa. The samples are centrifuged at a speed varying between 1000-15000 x g for 5-30 min and the retentate is collected and contains the majority of the N-terminal truncated Aβ protofibrils/oligomers.

### -HNE-modified N-terminal truncated Aβ protofibrils/oligomers

In a typical experiment 140 µM human wild-type N-terminal truncated Aβ3(pE)-42 is incubated with 5.6 mM HNE (e.g. with a ratio of 40:1 between HNE and Aβ3(pE)-42) for 20 hours at 37°C after which the excess of unbound HNE is removed using either Zeba desalt spin columns (Pierce Biotechnology, Rockford, IL, USA), Vivaspin 500 centrifugal device (Sartorius, Goettingen, Germany) or a Microcon centrifugal filter device (Millipore, Billerica, MA) according to the manufacturer's instructions. After this initial HNE-modification step, samples are analyzed directly. Prior to SEC-HPLC analysis, all samples are subjected to centrifugation at 16.900 x g for 5 min. at 22°C and only the soluble fraction is analyzed by SEC-HPLC using a Superose 6 PC3.2/30 column. The HNE stabilized Aβ3(pE)-42 protofibrils/oligomers elute in a peak at about 10-15 min.

### - ONE-modified Aβ3(pE)-42

In a typical experiment human wild-type Aβ3(pE)-42 (140 µM) is incubated with 4.2 mM ONE (e.g. with a ratio of 40:1 between ONE and Aβ3(pE)-42) for 20 hours at 37°C and the excess of unbound ONE is removed using Zeba desalt spin columns (Pierce Biotechnology, Rockford, IL, USA), Vivaspin 500 centrifugal device (Sartorius, Goettingen, Germany) or a Microcon centrifugal filter device (Millipore, Billerica, MA) according to the manufacturer's instructions. Prior to SEC-HPLC analysis, all samples are subjected to centrifugation at 16.900 x g for 5 min. at 22°C and only the soluble fraction is analyzed by SEC-HPLC using a Superose 6 PC3.2/30 column. ONE stabilized Aβ3(pE)-42 protofibrils/oligomers elute as the main peak at about 10-15 min.

### -HNE- and ONE-modified Aβ3(pE)-42

In a typical experiment human wild-type Aβ3(pE)-42 (140 µM) is incubated with 4.2 mM HNE and 4.2 mM ONE for 20 hours at 37°C and the excess of unbound HNE and ONE is removed using Zeba desalt spin columns (Pierce Biotechnology, Rockford, IL, USA), Vivaspin 500 centrifugal device (Sartorius, Goettingen, Germany) or a Microcon centrifugal filter device (Millipore, Billerica, MA) according to the manufacturer's instructions. All samples are subjected to centrifugation at 16.900 x g for 5 min. at 22°C and only the soluble fraction is analyzed by SEC-HPLC using a Superose 6 PC3.2/30 column. HNE and ONE stabilized Aβ3(pE)-42 protofibrils/oligomers elute as the main peak at about 10-15 min.

### Example 4. Aβ3(pE)-42 antibodies in mice immunized with N-terminal truncated protofibrils/oligomers

### -Immunization/Polyclonal antibodies

In the immunization scheme BALB/c mice are utilized. For the initial immunizations (e.g. 3-6 times), mice are injected with 30-50 µg of Aβ3(pE)-42 protofibrillar/oligomer preparations diluted in phosphate-buffered saline (PBS) + 0.1 % Tween 20 together with 5 µl ISCOM. For the final immunization, mice are injected with 30-50 µg Aβ3(pE)-42 protofibrillar/oligomer preparations without ISCOM. Plasma from immunized mice are analyzed for reactivity towards Aβ3(pE)-42 protofibrils/oligomers. The specificity of the polyclonal antibody response is analyzed by ELISA. In a typical experiment, a flat bottom high binding 96-well polystyrene microtiter plate is coated with an Aβ reactive antibody, the wells are blocked with PBS+0.05% Tween-20 and thereafter Aβ3(pE)-42 monomers or Aβ3(pE)-42 protofibrils/oligomers are diluted in PBS/0.1 % BSA/0.05 % Tween-20 and added at a final concentration of 1 ng/well. Plasma samples from immunized mice (taken at different times during the immunization schedule) are diluted in PBS/0.1 % BSA/0.05 % Tween-20 and added to wells. Horseradish-peroxidase (HRP)-conjugated goat anti-mouse IgG-antibody (Southern Biotech) is used as the secondary antibody at a dilution of 1/10000. Immunoreactivity is visualized using TMB (Neogen Corp.).

In the serum, antibodies that specifically recognize Aβ3(pE)-42 protofibrils/oligomers are detected. Similar ELISAs testing as described above are performed with other truncated or non-truncated forms of Aβ42, both as monomers and as protofibrils/oligomers, to evaluate the specificity of the polyclonal response.

### - Hybridoma/monoclonal antibodies

Spleen cells are isolated and grinded in sterile phosphate-buffered saline (PBS) and centrifuged at 1200 x g for 10 min to collect a cell-rich pellet. The cells are further washed with PBS and centrifuged at 1200 x g for 10 min. The cell pellet is resuspended in Dulbecco's minimum essential medium (DMEM, Invitrogen, La Jolla, CA, USA) supplemented with 1% antibiotics. Spleen cells are mixed at a 1:2 ratio with Sp2/0 cells (mouse myeloma cell line) in DMEM. To facilitate cell fusion, 1 ml of polyethylene glycol (Sigma-Aldrich, St. Louis, MO, USA) is added to the cell mixture and the reaction is stopped with the addition of DMEM. Cells are harvested and the pellet is resuspended in DMEM supplemented with 10% (v/v) fetal bovine serum (Cambrex, Charles City, IA, USA) and also containing HAT Media Supplement Hybri-Max™ (Sigma-Aldrich, St. Louis, MO, USA), 10% (v/v) BM condition media (Roche Diagnostics Scandinavia, Bromma, Sweden), 1% (v/v) sodium pyruvate (Cambrex, Charles City, IA, USA), 1% (v/v) antibiotics (Sigma-Aldrich, St. Louis, MO, USA) and 1% (v/v) L-glutamine (Cambrex, Charles City, IA, USA) and cells are plated on 96 well cell culturing plates.

To screen for Aβ3(pE)-42 protofibrillar/oligomer reactive antibodies produced by the generated hybridomas, an ELISA protocol is used. In a typical experiment, a flat bottom high binding 96-well polystyrene microtiter plate is coated with an Aβ reactive antibody, the wells are blocked with PBS+0.05% Tween-20 and thereafter Aβ3(pE)-42 monomers or Aβ3(pE)-42 protofibrils/oligomers are diluted in PBS/0.1 % BSA/0.05 % Tween-20 and added at a final concentration of 1 ng/well. Cell culture supernatants from the hybridomas are added to the wells. Horseradish-peroxidase (HRP)-conjugated goat anti-mouse IgG antibody (Southern Biotech) is used as the secondary antibody at a dilution of 1/10000. Immunoreactivity is visualized using an enhanced K-Blue® substrate (TMB). Aβ3(pE)-42 reactive hybridoma clones are selected and are further sub-cloned using Limited Dilution Assay (LDA).

Similar ELISAs as described above are performed with other truncated or non-truncated forms of Aβ42, both as monomers and as protofibrils/oligomers, to evaluate the specificity of the monoclonal antibodies produced by the hybridomas.

Hybridomas are generated by injecting Aβ3(pE)-42 protofibrillar/oligomer preparations as previously described. Other forms of truncated Aβ42 protofibrillar/oligomers as well as truncated Aβ42 protofibrillar/oligomeric preparations modified with HNE and/or ONE, or other aldehydes are also used as antigens to develop monoclonal antibodies binding other forms of truncated Aβ42 protofibrillar/oligomer and are tested using ELISA screening as described above.

Binding data from tests with antibodies according to the invention demonstrate high affinity for Aβ3(pE)-42 monomers as well as protofibrils/oligomers comprising 100% Aβ3(pE)-42, and with substantially no binding to full length Aβ1-42 monomers. This is in clear contrast to prior art antibodies which may exhibit high affinity for all these species or may bind protofibrils comprising only full length Aβ efficiently, but with substantially no affinity for N-terminal truncated Aβ forms.

The specific examples and embodiments described herein are exemplary only in nature and are not intended to be limiting of the invention defined by the claims.

### REFERENCES

Bacskai et al., Nat.Med. 7:369-372, 2001.
Bard et al., Nat. Med. 6:916-919, 2000.
Bayer et al., Neurology 64:94-101, 2005.
Brendza et al., J. Clin. Invest. 115:428-33, 2005.
Chen et al., Nature, 408:975-9, 2000.
Ding J.D. et al., Vision Res. 48:339-45, 2008.
Duncan & Winter, Nature, 332: 738-470, 1988
Englund H. et al., J of Neurochemistry, 103: 334-345, 2007
Ester W.P. Science 293, 1449-1459, 2001.
Guo L. et al., Proc. Natl Acad Sci, 104:13444-9, 2007.
Gullberg et al., Proc. Natl Acad Sci, 101:8420-4, 2004.
Hansen L, et al., Neurology 40, 1-8, 1990.
Hoshi ct al. Proc. Natl Acad. Sci, 100:6370-6375, 2003.
Idusogie et al, Journal of Immunology, 2000, 164: 4178-84
Jarret J.T. , Biochemistry, 32, 4693-4697,1993.
Jellinger K.H. et al., J Neurol Sci. 257(1-2):80-7, 2007
Johnson L.V. et al., Proc. Natl Acad Sci 99:11830-35, 2002
Leatherbarrow R.J. et al., Mol. Immunol. 22, 407, 1985.
Lord et al., Neurobiol. Aging, 27:67-77, 2006.
McLean et al., Ann. Neurol. 46:860-866, 1999.
Mullan et al., Nat Genet. 1:345-347, 1992.
Needham M and Mastaglia FL., Lancet Neurol 6: 620-31, 2007.
Nilsberth et al., Nat Neurosci. 4:887-893, 2001.
Näslund et al., JAMA, 283:1571-1577, 2000.
Pfeifer et al., Science 298:1379, 2002.
Racke et al., J.Neurosci 25 :629-36, 2005.
Schenk D. et al. Nature, 400, 173-177, 1999.
Sergeant et al., J Neurochem, 85, 1581-1591, 2003
Stenh et al., Ann. Neurol. 58:147-50, 2005.
Walsh D. M. et al., 272, 22364-22372,1997
Walsh D. M.et al., Nature, 416, 535-9, 2002.
Wilcock et al., J. Neurosci., 23:3745-51, 2003.
Wright A. et al., J. of Immunology, 3393-3402, 1998.
Xu Y. et al. J.Biol. Chem. 269, 3469-3474,1994
Zigman et al.,J Mental Retardation and Developmental Disabilities Research Reviews 2: 73-79, 1996.

## Claims

1. An antibody, wherein the antibody is raised against stabilized protofibril comprising the N-terminal truncated Amyloid beta (Aβ) Aβ3(pE)-42, and is selected such that it (i) binds soluble protofibril comprising Aβ3(pE)-42, (ii) binds Aβ3(pE)-42 monomer, and (iii) exhibits no or substantially no binding with non-N-terminal truncated Aβ monomer, wherein:
(a) the non-N-terminal truncated Aβ monomer may or may not have a C-terminal truncation; and
(b) the stabilized protofibril is stabilized with a hydrophobic organic agent comprising a non-ionic detergent, a zwitterionic detergent, 4-hydroxy-2-nonenal (HNE), or 4-oxo-2-nonenal (ONE), and wherein the stabilized protofibril has a lower formation rate to a non-soluble aggregated form than a non-stabilized form of the protofibril.

2. The antibody according to claim 1, wherein the soluble protofibril to which the antibody binds comprises Aβ comprising the Arctic mutation (E22G), the Dutch mutation (E22Q), the Italian mutation (E22K), the Iowa mutation (D23N), or the Flemish mutation (A21G), or combinations of two or more thereof.

3. The antibody according to claim 1 or 2 which binds to a protofibril comprising at least 80% Aβ3(pE)-42 with an IC50 value less than or equal to 25 nM

4. The antibody according to claim 3 which binds to a protofibril comprising 80 % Aβ3(pE)-42 and 20 % Aβ1-42 with an IC50 value less than or equal to 25 nM.

5. The antibody of claim 1 which binds a protofibril comprising 100% Aβ3(pE)-42 with an IC50 value of less than or equal to 100 nM.

6. The antibody according to any one of claims 1-5 wherein the antibody is human, humanized, or modified to reduce antigenicity in human, and/or wherein the antibody has reduced complement activity, and/or wherein the antibody is a Fab fragment, e.g. F(ab), F(ab)2 or DiFabody.

7. The antibody according to any one of claims 1-6, wherein the antibody is monoclonal.

8. The antibody according to any one of claims 1-7, wherein the antibody also binds the stabilized protofibril.

9. A method for detecting protofibril comprising N-terminal truncated Aβ3(pE)-42 and for detecting N-terminal truncated Aβ3(pE)-42 monomer, comprising adding an antibody according to any one of claims 1-8 to a biological sample comprising or suspected of comprising the protofibril and/or monomer, and measuring a concentration of a complex formed between the antibody and the protofibril and a complex formed between the antibody and the monomer.

## Patentansprüche

1. Antikörper, wobei der Antikörper gegen eine stabilisierte Protofibrille erzeugt wird, umfassend das N-terminale verkürzte Amyloid-beta (Aβ) Aβ3(pE)-42, und so ausgewählt ist, dass er (i) eine lösliche Protofibrille bindet, die Aβ3(pE)-42 umfasst, (ii) das Aß3(pE)-42-Monomer bindet und (iii) keine oder im Wesentlichen keine Bindung mit dem nicht-N-terminalen verkürzten Aß-Monomer zeigt, wobei
(a) das nicht-N-terminale verkürzte Aß-Monomer eine C-terminale Verkürzung aufweisen kann oder nicht; und
(b) die stabilisierte Protofibrille mit einem hydrophoben organischen Mittel stabilisiert ist, das ein nicht-ionisches Detergens, ein zwitterionisches Detergens, 4-Hydroxy-2-nonenal (HNE) oder 4-Oxo-2-nonenal (ONE) umfasst, und wobei die stabilisierte Protofibrille eine geringere Bildungsrate zu einer nicht-löslichen aggregierten Form als eine nicht-stabilisierte Form der Protofibrille aufweist.

2. Antikörper nach Anspruch 1, wobei die lösliche Protofibrille, an das der Antikörper bindet, Aβ umfasst, umfassend die arktische Mutation (E22G), die niederländische Mutation (E22Q), die italienische Mutation (E22K), die Iowa-Mutation (D23N) oder die flämische Mutation (A21G) oder Kombinationen von zwei oder mehreren davon.

3. Antikörper nach Anspruch 1 oder 2, der an eine Protofibrille, die mindestens 80% Aβ3(pE)-42 umfasst, mit einem IC50-Wert von kleiner oder gleich 25 nM bindet.

4. Antikörper nach Anspruch 3, der an eine Protofibrille, die 80% Aβ3(pE)-42 und 20% Aβ1-42 umfasst, mit einem IC50-Wert von kleiner oder gleich 25 nM bindet.

5. Antikörper nach Anspruch 1, der an eine Protofibrille, die 100% Aβ3(pE)-42 umfasst, mit einem IC50-Wert von kleiner oder gleich 100 nM bindet.

6. Antikörper nach einem der Ansprüche 1-5, wobei der Antikörper human, humanisiert oder modifiziert ist, um die Antigenizität beim Menschen zu reduzieren, und/oder wobei der Antikörper eine reduzierte Komplementaktivität aufweist und/oder wobei der Antikörper ein Fab-Fragment, z. B. F(ab), F(ab)2 oder DiFabody, ist.

7. Antikörper nach einem der Ansprüche 1-6, wobei der Antikörper monoklonal ist.

8. Antikörper nach einem der Ansprüche 1-7, wobei der Antikörper auch die stabilisierte Protofibrille bindet.

9. Verfahren zum Nachweis eines Protofibrills, das N-terminale verkürzte Aβ3(pE)-42 enthält, und zum Nachweis des N-terminalen verkürzten Aß3(pE)-42-Monomers, was die Zugabe eines Antikörpers nach einem der Ansprüche 1-8 zu einer biologischen Probe umfasst, welche die Protofibrille und/oder das Monomer enthält oder von der vermutet wird, dass sie die Protofibrille und/oder das Monomer enthält, und zum Messen der Konzentration eines Komplexes, der zwischen dem Antikörper und der Protofibrille gebildet wird, und eines Komplexes, der zwischen dem Antikörper und dem Monomer gebildet wird.

## Revendications

1. Anticorps, l'anticorps étant dirigé contre une protofibrille stabilisée comprenant le bêta amyloïde (Aβ) Aβ3(pE)-42 tronqué à l'extrémité N-terminale, et étant choisi de façon à ce qu'il (i) se lie à une protofibrille soluble comprenant l'Aβ3(pE)-42, (ii) se lie à un monomère d'Aβ3(pE)-42, et (iii) ne révèle aucune ou pratiquement aucune liaison avec un monomère d'Aβ non tronqué à l'extrémité N-terminale, dans lequel :
(a) le monomère d'Aβ non tronqué à l'extrémité N-terminale peut avoir ou ne pas avoir une troncature à l'extrémité C-terminale ; et
(b) la protofibrille stabilisée est stabilisée au moyen d'un agent organique hydrophobe comprenant un détergent non ionique, un détergent zwitterionique, du 4-hydroxy-2-nonénal (HNE), ou du 4-oxo-2-nonénal (ONE), et dans lequel la protofibrille stabilisée présente une vitesse de formation en une forme agrégée non soluble inférieure à celle d'une forme de la protofibrille non stabilisée.

2. Anticorps selon la revendication 1, dans lequel la protofibrille soluble auquel l'anticorps se lie comprend l'Aβ comprenant la mutation Arctique (E22G), la mutation Néerlandaise (E22Q), la mutation Italienne (E22K), la mutation de l'Iowa (D23N), ou la mutation Flamande (A21G), ou des combinaisons de deux ou plus de celles-ci.

3. Anticorps selon la revendication 1 ou 2 qui se lie à une protofibrille comprenant au moins 80 % d'Aβ3(pE)-42 avec une valeur CI50 inférieure ou égale à 25 nM.

4. Anticorps selon la revendication 3 qui se lie à une protofibrille comprenant 80 % d'Aβ3(pE)-42 et 20 % d'Aβ1-42 avec une valeur CI50 inférieure ou égale à 25 nM.

5. Anticorps selon la revendication 1 qui se lie à une protofibrille comprenant 100 % d'Aβ3(pE)-42 avec une valeur CI50 inférieure ou égale à 100 nM.

6. Anticorps selon l'une quelconque des revendications 1 à 5, l'anticorps étant humain, humanisé, ou modifié pour réduire l'antigénicité chez l'Homme, et/ou l'anticorps ayant une activité complémentaire réduite, et/ou l'anticorps étant un fragment Fab, par ex. F(ab), F(ab)2 ou DiFabody.

7. Anticorps selon l'une quelconque des revendications 1 à 6, l'anticorps étant monoclonal.

8. Anticorps selon l'une quelconque des revendications 1 à 7, l'anticorps se liant aussi à la protofibrille stabilisée.

9. Procédé de détection d'une protofibrille comprenant l'Aβ3(pE)-42 tronqué à l'extrémité N-terminale et de détection d'un monomère d'Aβ3(pE)-42 tronqué à l'extrémité N-terminale, comprenant l'ajout d'un anticorps selon l'une quelconque des revendications 1 à 8 à un échantillon biologique comprenant ou suspecté de comprendre la protofibrille et/ou le monomère, et la mesure d'une concentration d'un complexe formé entre l'anticorps et la protofibrille et d'un complexe formé entre l'anticorps et le monomère.
